# EUROPEAN PATENT APPLICATION

(11) **EP 4 092 046 A1**
(43) Date of publication of application: **23.11.2022**
(21) Application number: 21740659.4
(22) Date of filing: 13.01.2021
(51) Int. Cl.: C07K 14/50, C12N 5/02, C12N 5/074

(54) **CELL CULTURE MEDIUM COMPOSITION**

(30) Priority: 14.01.2020 JP 2020003958
(71) Applicant: AJINOMOTO CO., INC., Chuo-ku Tokyo 104-8315 (JP)
(72) Inventor: OGAWA, Shimpei, Kawasaki-shi, Kanagawa 210-8681 (JP); HIGUCHI, Takuya, Kawasaki-shi, Kanagawa 210-8681 (JP); FUROMITSU, Shumpei, Kawasaki-shi, Kanagawa 210-8681 (JP); ITO, Kenichiro, Kawasaki-shi, Kanagawa 210-8681 (JP); NISHIYAMA, Megumi, Kawasaki-shi, Kanagawa 210-8681 (JP)
(74) Representative: Hoffmann Eitle
(86) International application number: PCT/JP2021/000744
(87) International publication number: WO 2021/145319

(57) **Abstract**

A medium composition for cell culture, containing a basic fibroblast growth factor (bFGF) at not less than 150 ng/mL is provided by the present invention.

## Description

### [Technical Field]

The present invention relates to a medium composition for cells. More particularly, the present invention relates to a medium composition for high density suspension culture of cells.

### [Background Art]

Regenerative medicine technology using cells has been receiving high attention in recent years as one of the means capable of treating various diseases and damages that were difficult to treat before. Since regenerative medicine requires a large amount of cells, the development of a method for efficiently culturing cells has been actively conducted. For example, Patent Literature 1 reports a method for culturing stem cells, including treating stem cells with a ROCK inhibitor in a medium. In addition, the present inventors also reported in 2018 a high density culture method of animal cells, including adding glucose and/or specific amino acids to a medium, and the like (Patent Literature 2).

### [Citation List]

### [Patent Literature]

[PTL 1]
   JP-A- 2008-099662
[PTL 2]
   Japanese Patent Application No. 2018-184352

### [Summary of Invention]

### [Technical Problem]

The method described in Patent Literature 2 is one of the very superior methods for high density culture of animal cells including pluripotent stem cells. This time, the present inventors reconsidered the method and attempted to develop a more preferable high density culture method of cells.

### [Solution to Problem]

The present inventors have conducted intensive studies of the above-mentioned problems and found that cell proliferation is more remarkably promoted in high density culture including suspension culture of cells, by adding basic fibroblast growth factor (bFGF) to the medium in an amount far higher than that previously recommended in the pertinent technical field. In addition, they have also found that, when the cells are pluripotent stem cells, the addition of large amounts of bFGF can maintain the undifferentiated state of the pluripotent stem cells to be subjected to high density culture extremely well, and that pluripotent stem cells prepared by high density culture in a medium supplemented with a large amount of bFGF have extremely good differentiation potency. Based on such findings, they have conducted further studies and completed the present invention.

Accordingly, the present invention provides the following.
[1] A medium composition for cell culture, comprising a basic fibroblast growth factor (bFGF) at not less than 150 ng/mL, or a stabilized bFGF at a concentration that affords an effect equivalent to that of bFGF at said concentration.
[2] The medium composition of [1], wherein the composition is for use in suspension culture.
[3] The medium composition of [1] or [2], wherein the composition is for use in high density culture.
[4] The medium composition of [3], wherein cells to be subjected to suspension culture have a cell density of not less than 6.0×10⁵ cells/mL.
[5] The medium composition of any of [1] to [4], wherein the cell is a pluripotent stem cell, an adult stem cell, or a progenitor cell.
[6] A method for culturing a cell, comprising suspension culture of the cell in the medium composition of [1].
[7] The method of [6], wherein the medium comprises bFGF at 10 to 1000 ng/mL/day, or stabilized bFGF at a concentration that affords an effect equivalent to that of bFGF at said concentration.
[8] The method of [6] or [7], wherein pluripotent stem cells to be subjected to suspension culture have a cell density of not less than 6.0×10⁵ cells/mL.
[9] The method of any of [6] to [8], wherein the cell is a pluripotent stem cell, an adult stem cell, or a progenitor cell.

### [Advantageous Effects of Invention]

According to the present invention, cells can be prepared extremely efficiently. According to the present invention, moreover, high quality pluripotent stem cells that maintain an undifferentiated state well and have good differentiation potency can be prepared highly efficiently.

### [Brief Description of Drawings]

[Fig. 1]

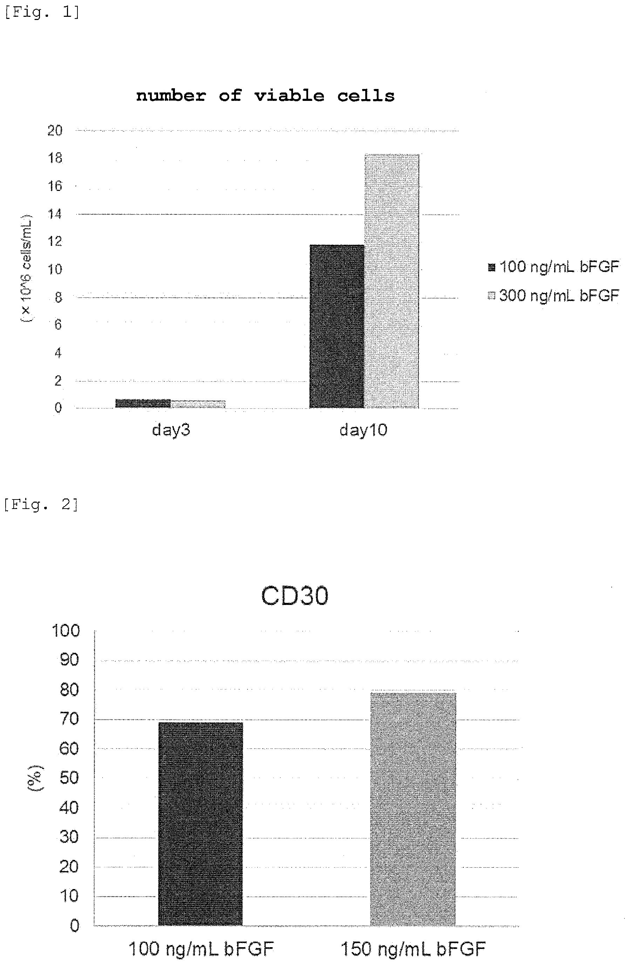

   Fig. 1 shows the influence of the bFGF concentration on the proliferation of iPS cells (n=1).
[Fig. 2]
   Fig. 2 shows the influence of the amount of addition of bFGF on the expression of CD30 in iPS cells (n=1).
[Fig. 3]

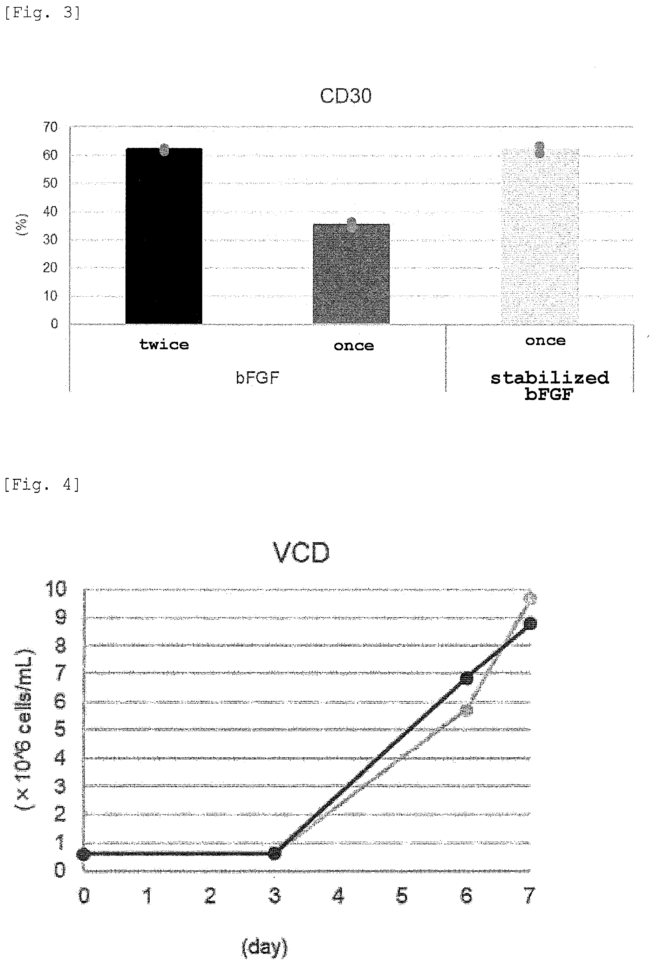

   Fig. 3 shows that the desired effect of the present invention can also be obtained by using stabilized bFGF.
[Fig. 4]
   Fig. 4 shows the time-course changes of the cell number (VCD: Viable Cell Density) when iPS cells were cultured in a medium containing high concentration bFGF.
[Fig. 5]

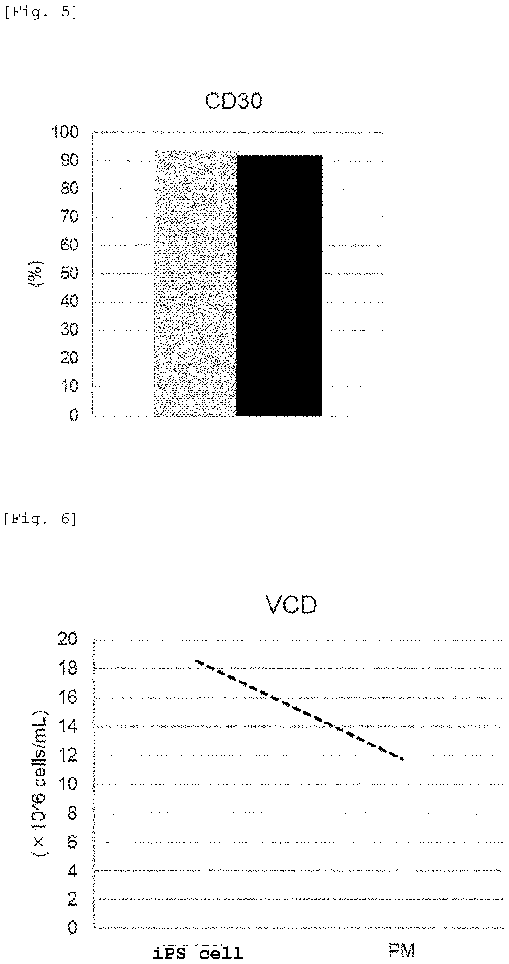

   Fig. 5 shows the expression level of CD30 in iPS cells cultured in a medium containing high concentration bFGF.
[Fig. 6]
   Fig. 6 shows that the iPS cells cultured in a medium containing high concentration bFGF could be highly efficiently differentiated in a high density state exceeding 1.0×10⁷ cells/mL into paraxial mesoderm (PM).
[Fig. 7]

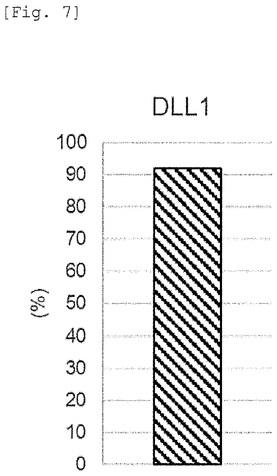

   Fig. 7 shows that the cells induced to differentiate from iPS cells cultured in a medium containing high concentration bFGF highly express DLL1 (PM marker).

### [Description of Embodiments]

The present invention is explained in detail in the following.

### Definition

In the present specification, the "suspension culture" refers to a cell culture method performed in a state where cells do not adhere to the culture container. In the present invention, the suspension culture may or may not be accompanied by pressure from the outside or vibration on the liquid medium, or shaking or rotation operation in the liquid medium.

In the present specification, the "high density culture" refers to a culture at a high cell density compared to the cell density expected in general cell culture. The criteria for high density may vary depending on the culture method (contact culture/suspension culture, etc.), cell type, and the like. For example, in the case of suspension culture of iPS cells, culture at a density of not less than 6×10⁵ cells/mL (preferably, not less than 2×10⁶ cells/mL) is defined as the high density culture in the present specification.

In the present specification, the "pluripotent stem cell" means a cell capable of differentiating into any tissue or cell constituting living organisms. Examples of the pluripotent stem cell include, but are not limited to, embryonic stem cell (ES cell), embryonic germ cell (EG cell), induced pluripotent stem cell (iPS cell), and the like.

In the present specification, the "adult stem cell (also called somatic stem cell)" means a cell that has the ability to differentiate into a cell constituting a specific tissue (organ). Examples of the adult stem cell include, but are not limited to, hematopoietic stem cell, neural stem cell, germ stem cell, intestinal stem cell, epidermis stem cell, mesenchymal stem cell, and the like.

In the present specification, the "progenitor cell" means a cell in the process of differentiating from the aforementioned pluripotent stem cell or adult stem cell into a specific somatic cell or reproductive cell.

### 1. medium composition

The present invention provides a medium composition for cell culture, containing basic fibroblast growth factor (bFGF) at a concentration of not less than 150 ng/mL, or a stabilized bFGF at a concentration that affords an effect equivalent to that of bFGF at said concentration (hereinafter sometimes referred to as "the medium composition of the present invention").

The medium composition of the present invention can be prepared by adding bFGF at a very high concentration to a general basal medium for cells.

The medium for cells that is used for preparing the medium composition of the present invention may be prepared by a method known per se according to the cells to be cultured, or a commercially available product.

Examples of the commercially available medium include Dulbecco's modified Eagle medium (DMEM), Ham's Nutrient Mixture F12, DMEM/F12 medium, McCoy's 5A medium, Minimum Essential medium (MEM), Eagle's Minimum Essential medium (EMEM), alpha Modified Eagle's Minimum Essential medium (aMEM), Roswell Park Memorial Institute (RPMI) 1640 medium, Iscove's Modified Dulbecco's medium (IMDM), MCDB131 medium, William's medium E, Fischer's medium, and the like.

Examples of the medium particularly for stem cell culture include STEMPRO (registered trade mark) hESC SFM medium (Life Technologies), mTeSR1 medium (STEMCELL Technologies), TeSR2 medium (STEMCELL Technologies), TeSR-E8 medium (STEMCELL Technologies), Essential 8 medium (Life Technologies), HEScGRO (trade mark) Serum-Free medium for hES cells (Millipore), PluriSTEM (trade mark) Human ES/iPS medium (EMD Millipore), NutriStem (registered trade mark) hESC XF medium (Biological Industries Israel Beit-Haemek), NutriStem (trade mark) XF/FF Culture medium (Stemgent), AF NutriStem (registered trade mark) hESC XF medium (Biological Industries Israel Beit-Haemek), S-medium (DS pharma biomedical), StemFit (registered trade mark) AK03N medium (Ajinomoto Co., Inc.), hESF9 medium, hESF-FX medium, CDM medium, DEF-CS 500 Xeno-Free 3D Spheroid Culture medium (Cellartis), StemFlex medium (Thermo Fisher Scientific), and the like.

At the time of filing the present application, the amount of bFGF contained in the medium for culturing cells is about 100 ng/mL at most. This is because the effect on cell proliferation and/or undifferentiation is considered to be constant even when bFGF is added at a higher concentration. The lower limit of the concentration of bFGF contained in the medium composition of the present invention is generally 150 ng/mL, preferably 200 ng/mL, more preferably 250 ng/mL, further preferably 275 ng/mL, particularly preferably 300 ng/mL, or may be higher. The upper limit is not particularly set, and may be generally 1500 ng/mL, preferably 1000 ng/mL, more preferably 800 ng/mL, further preferably 600 ng/mL, particularly preferably 500 ng/mL, from the aspects of the cost and the like. In one embodiment, the concentration of bFGF in the medium composition of the present invention may be generally 150 to 1500 ng/mL, preferably 200 to 1000 ng/mL, more preferably 250 to 800 ng/mL, further preferably 275 to 600 ng/mL, particularly preferably 300 to 500 ng/mL.

In one embodiment, bFGF to be added to the medium composition of the present invention may be a stabilized bFGF. The stabilized bFGF that may be added to the medium composition of the present invention may be produced by a method known per se, or may be a commercially available product. Examples of the preferred commercially available product include, but are not limited to, Heat Stable Recombinant Human bFGF (manufactured by Thermo Fisher Scientific) and the like. As other one embodiment of the stabilized bFGF, "FGF2-G3" can be mentioned. FGF2-G3 is a variant bFGF protein having nine amino acid mutations (R31L, V52T, E54D, H59F, S94I, L92Y, C96N, S109E, T121P) as compared with wild-type bFGF. Without being bound by theory, FGF2-G3 was reported to show activities equivalent to those of unstabilized wild-type bFGF even when used in the amount of about 40% compared with the unstabilized one (Hui-Hsuan Kuo et al., Negligible-Cost and Weekend-Free Chemically Defined Human iPSC Culture).

In this embodiment, a preferred concentration range of the stabilized bFGF may be a concentration that affords a cell proliferation promoting effect and/or an undifferentiated state maintaining effect equivalent to those/that of unstabilized bFGF. When stabilized bFGF is used, a preferred concentration range can be appropriately set by those of ordinary skill in the art by confirming the difference in the effect between the unstabilized bFGF and the stabilized bFGF by a method known per se, by referring to the above-mentioned concentration range. The concentration of stabilized bFGF in a medium composition may vary depending on the means and degree of stabilization of bFGF. Generally, a concentration range that affords an effect equivalent to the desired effect of the present invention (cell proliferation promoting effect and/or undifferentiated state maintaining effect) that is achieved by adopting the concentration range of the aforementioned unstabilized bFGF may be appropriately set. Specifically, the concentration range of the stabilized bFGF may be determined as follows:
(1) the effect (that is, the cell proliferation promoting effect and/or the undifferentiated state maintaining effect) when a medium composition containing unstabilized bFGF is used is quantified (the effect can be quantified by the method used in Examples described later of the present application),
(2) using stabilized bFGF instead of unstabilized bFGF, the same test as in (1) is performed, and the concentration of stabilized bFGF that affords an effect equivalent to the effect quantified in (1) is determined.

In one embodiment, the lower limit of the concentration of stabilized bFGF contained in the medium composition of the present invention is generally 75 ng/mL, preferably 100 ng/mL, more preferably 125 ng/mL, further preferably 138 ng/mL, particularly preferably 150 ng/mL, or may be higher. The upper limit is not particularly set, and may be generally 750 ng/mL, preferably 500 ng/mL, more preferably 400 ng/mL, further preferably 300 ng/mL, particularly preferably 250 ng/mL, from the aspects of the cost and the like. In one embodiment, the concentration of bFGF in the medium composition of the present invention may be generally 75 to 750 ng/mL, preferably 100 to 500 ng/mL, more preferably 125 to 400 ng/mL, further preferably 138 to 300 ng/mL, particularly preferably 150 to 250 ng/mL.

In one embodiment, the lower limit of the concentration of stabilized bFGF contained in the medium composition of the present invention is generally 50 ng/mL, preferably 67 ng/mL, more preferably 84 ng/mL, further preferably 92 ng/mL, particularly preferably 100 ng/mL, or may be higher. The upper limit is not particularly set, and may be generally 500 ng/mL, preferably 334 ng/mL, more preferably 267 ng/mL, further preferably 200 ng/mL, particularly preferably 167 ng/mL, from the aspects of the cost and the like. In one embodiment, the concentration of bFGF in the medium composition of the present invention may be generally 50 to 500 ng/mL, preferably 67 to 334 ng/mL, more preferably 84 to 267 ng/mL, further preferably 92 to 200 ng/mL, particularly preferably 100 to 167 ng/mL.

In one embodiment, the lower limit of the concentration of stabilized bFGF contained in the medium composition of the present invention is generally 38 ng/mL, preferably 50 ng/mL, more preferably 63 ng/mL, further preferably 69 ng/mL, particularly preferably 75 ng/mL, or may be higher. The upper limit is not particularly set, and may be generally 375 ng/mL, preferably 250 ng/mL, more preferably 200 ng/mL, further preferably 150 ng/mL, particularly preferably 125 ng/mL, from the aspects of the cost and the like. In one embodiment, the concentration of bFGF in the medium composition of the present invention may be generally 38 to 375 ng/mL, preferably 50 to 250 ng/mL, more preferably 63 to 200 ng/mL, further preferably 69 to 150 ng/mL, particularly preferably 75 to 125 ng/mL.

Alternatively, bFGF can also be stabilized by separately adding a compound that contributes to the stabilization of bFGF to the medium. One such embodiment is, for example, addition of a sulfated compound (e.g., sulfated polysaccharides such as sodium dextran sulfate and the like, sulfated polymers such as sulfo group-containing polyvinyl alcohol and the like, sugar lactone sulfides such as gluconolactone-SO₃NA and the like, etc.) (refer to WO2013/147264 for details). When bFGF is stabilized in such method, those of ordinary skill in the art can appropriately determine the concentration range of bFGF in consideration of the aforementioned concentration range of unstabilized bFGF, the degree of stabilization of bFGF, and the effect to be achieved.

In addition to the above, components preferable for cell proliferation can be further added to the medium composition of the present invention. Examples of such component include sugars such as glucose, fructose, sucrose, maltose, and the like; amino acids such as asparagine, aspartic acid, glutamine, glutamic acid, and the like; proteins such as albumin, transferrin, and the like; peptides such as glycylglycylglycine, soybean peptide, and the like; serum; vitamins such as choline, vitamin A, vitamin Bs (thiamine, riboflavin, pyridoxine, cyanocobalamin, biotin, folic acid, pantothenic acid, nicotine amide etc.), vitamin C, vitamin E, and the like; fatty acids such as oleic acid, arachidonic acid, linoleic acid, and the like; lipids such as cholesterol and the like; inorganic salts such as sodium chloride, potassium chloride, calcium chloride, magnesium sulfate, sodium dihydrogen phosphate, and the like; trace elements such as zinc, copper, selenium, and the like; buffering agents such as N,N-bis(2-hydroxyethyl)-2-aminoethanesulfonic acid (BES), 4-(2-hydroxyethyl)-1-piperazineethanesulfonic acid (HEPES), N-[tris(hydroxymethyl)methyl]glycine (Tricine), and the like; antibiotics such as amphotericin B, kanamycin, gentamicin, streptomycin, penicillin, and the like; cell adhesion factors and extracellular matrix components such as Type I collagen, Type II collagen, fibronectin, laminin, poly-L-lysine, poly-D-lysine, and the like; cytokines and growth factors such as interleukin, fibroblast growth factor (FGF), hepatocyte growth factor (HGF), transforming growth factor (TGF)-α, transforming growth factor (TGF)-β, vascular endothelium growth factor (VEGF), activin A, and the like; hormones such as dexamethasone, hydrocortisone, estra diol, progesterone, glucagon, insulin, and the like; and the like. Appropriate components can be selected and used according to the type of the cells to be cultured.

In one embodiment, the medium composition of the present invention may contain choline (or a salt thereof) at the following concentrations (amount converted to free form in the case of choline salt):
[1] 1 to 100 mg/L, 10 to 100 mg/L, 20 to 100 mg/L, 30 to 100 mg/L, 40 to 100 mg/L, 50 to 100 mg/L, 60 to 100 mg/L, 70 to 100 mg/L, 80 to 100 mg/L, 90 to 100 mg/L;
[2] 1 to 90 mg/L, 10 to 90 mg/L, 20 to 90 mg/L, 30 to 90 mg/L, 40 to 90 mg/L, 50 to 90 mg/L, 60 to 90 mg/L, 70 to 90 mg/L, 80 to 90 mg/L;
[3] 1 to 80 mg/L, 10 to 80 mg/L, 20 to 80 mg/L, 30 to 80 mg/L, 40 to 80 mg/L, 50 to 80 mg/L, 60 to 80 mg/L, 70 to 80 mg/L;
[4] 1 to 70 mg/L, 10 to 70 mg/L, 20 to 70 mg/L, 30 to 70 mg/L, 40 to 70 mg/L, 50 to 70 mg/L, 60 to 70 mg/L;
[5] 1 to 60 mg/L, 10 to 60 mg/L, 20 to 60 mg/L, 30 to 60 mg/L, 40 to 60 mg/L, 50 to 60 mg/L;
[6] 1 to 50 mg/L, 10 to 50 mg/L, 20 to 50 mg/L, 30 to 50 mg/L, 40 to 50 mg/L;
[7] 1 to 40 mg/L/, 10 to 40 mg/L, 20 to 40 mg/L, 30 to 40 mg/L;
[8] 1 to 30 mg/L/, 10 to 30 mg/L/, 20 to 30 mg/L;
[9] 1 to 20 mg/L, 10 to 20 mg/L).

In one preferred embodiment, D-glucose and five kinds of amino acids (tryptophan, serine, cysteine (or cystine), methionine, arginine) may be further added to the medium composition of the present invention. The amount of these components to be added can be appropriately set according to the purpose of the culture, various culture conditions (e.g., cell density, frequency of medium exchange), and the like, by referring to the corresponding description in "2. cell culture method" below.

In one embodiment, the cell may be, but is not limited to, a pluripotent stem cell, an adult stem cell, or a progenitor cell.

In one embodiment, the pluripotent stem cell may be an embryonic stem cell (ES cell) or an iPS cell, preferably an iPS cell. The adult stem cell may be, but is not limited to, a hematopoietic stem cell, a neural stem cell, a germ stem cell, an intestinal stem cell, an epidermis stem cell, or a mesenchymal stem cell. The origin of the pluripotent stem cell, adult stem cell, and progenitor cell is also not particularly limited, but those derived from mammals are preferred, and those derived from human are more preferred.

In one embodiment, the medium composition of the present invention may be provided as a medium for suspension culture of pluripotent stem cells. In another embodiment, the medium composition of the present invention may be provided as a medium for suspension culture of pluripotent stem cells and for high density culture. The medium composition of the present invention has a function of maintaining high density suspension culture of pluripotent stem cells in a preferable state, and can realize very good cell proliferation of pluripotent stem cells. At the same time, the pluripotent stem cells proliferated using the medium composition of the present invention maintain a good undifferentiated state. That is, the pluripotent stem cells proliferated using the medium composition of the present invention have high quality and therefore can be preferably used for, for example, regenerative medicine and the like. Considering this point, the medium composition of the present invention can be said to be a medium composition for undifferentiated state maintenance culture of pluripotent stem cells, or also a medium composition for maintaining an undifferentiated state and for promoting proliferation of pluripotent stem cells. The undifferentiated state of pluripotent stem cells may be confirmed by a method known per se. For example, those skilled in the art can easily confirm by detecting the expression of known markers (e.g., CD30, Oct3/4, Nanog, etc.) indicating an undifferentiated state.

The medium of the present invention may be provided in a liquid state, or in a state of being concentrated more than the concentration at the time of use, or in a solid state such as freeze-dried powder and the like to be diluted with a solvent such as water and the like when in use, or dissolved or dispersed in a solvent such as water and the like before use. Furthermore, since bFGF is easily decomposed thermally, it can also be added immediately before using the medium.

### 2. cell culture method

The present invention also provides a method for culturing a cell, including suspension culture of the cell in the medium composition of the present invention (hereinafter sometimes referred to as "the method of the present invention").

In one embodiment of the method of the present invention, bFGF is further added to a medium under culture. The amount of the bFGF to be further added is not particularly limited as long as the desired effect of the present invention is obtained. It may be generally 10 to 1000 ng/mL/day, preferably 30 to 800 ng/mL/day, more preferably 50 to 600 ng/mL/day, further preferably 70 to 500 ng/mL/day, particularly preferably 105 to 420 ng/mL/day.

In one embodiment of the present invention, stabilized FGF may also be further added. The amount of the stabilized bFGF to be further added may vary depending on the means and degree of stabilization of bFGF. Generally, an amount that affords an effect equivalent to the desired effect of the present invention (cell proliferation promoting effect and/or undifferentiated state maintaining effect) that is achieved by adopting the amount of the aforementioned unstabilized bFGF may be appropriately set.

In another embodiment of the method of the present invention, stabilized bFGF is further added to a medium under culture. The amount of the stabilized bFGF to be further added is not particularly limited as long as the desired effect of the present invention is obtained. It may be generally 5 to 500 ng/mL/day, preferably 15 to 400 ng/mL/day, more preferably 25 to 300 ng/mL/day, further preferably 35 to 250 ng/mL/day, particularly preferably 53 to 210 ng/mL/day.

In another embodiment of the method of the present invention, the amount of the stabilized bFGF to be further added is not particularly limited as long as the desired effect of the present invention is obtained. It may be generally 4 to 334 ng/mL/day, preferably 10 to 267 ng/mL/day, more preferably 17 to 200 ng/mL/day, further preferably 24 to 167 ng/mL/day, particularly preferably 35 to 140 ng/mL/day.

In another embodiment of the method of the present invention, the amount of the stabilized bFGF to be further added is not particularly limited as long as the desired effect of the present invention is obtained. It may be generally 3 to 250 ng/mL/day, preferably 8 to 200 ng/mL/day, more preferably 13 to 150 ng/mL/day, further preferably 18 to 125 ng/mL/day, particularly preferably 27 to 105 ng/mL/day.

In one embodiment of the method of the present invention, the aforementioned further addition of bFGF or stabilized bFGF can also be performed using the medium of the present invention at the timing of medium exchange. The frequency of medium exchange may be appropriately determined according to the cell density, cell type, and the like, and is not particularly limited. To prevent the concentration of active bFGF in the medium from decreasing to a level adversely affecting the proliferation and/or maintenance of an undifferentiated state of pluripotent stem cells, medium exchange is performed at least once a day (preferably at least twice or more), and at the time of the medium exchange, 50 to 100% (preferably 60 to 100%, 70 to 100%, 80 to 100%, 90 to 100%, or 100%) of the medium in use may be exchanged with the medium composition of the present invention. In one embodiment, the frequency of medium exchange is at least once (e.g., once, twice, or three times) a day, and 70 to 100% of the medium in use may be exchanged with the medium composition of the present invention at the time of the medium exchange.

In one embodiment, D-glucose and five kinds of amino acids (tryptophan, serine, cysteine (or cystine), methionine, arginine) may be further added to the medium of the present invention.

Glucose (or a salt thereof) can be added to the medium of the present invention such that the converted glucose concentration is generally 0.1 g/L/day to 900 g/L/day, preferably 1 g/L/day to 200 g/L/day, more preferably 1 g/L/day to 20 g/L/day.

In addition, five kinds of 5 amino acids (tryptophan, serine, cysteine (cystine), methionine, and arginine) can be added to the medium of the present invention such that the concentration of tryptophan (concentration after conversion to tryptophan in a free form) is generally 0.1 mg/L/day to 11000 mg/L/day, preferably 1 mg/L/day to 1000 mg/L/day, more preferably 1 mg/L/day to 100 mg/L/day, the concentration of serine (concentration after conversion to serine in a free form) is generally 0.1 mg/L/day to 425000 mg/L/day, preferably 1 mg/L/day to 1000 mg/L/day, more preferably 1 mg/L/day to 100 mg/L/day, the concentration of cysteine or cystine (concentration after conversion to cysteine in a free form) is generally 0.1 mg/L/day to 280000 mg/L/day, preferably 1 mg/L/day to 1000 mg/L/day, more preferably 1 mg/L/day to 100 mg/L/day, the concentration of methionine (concentration after conversion to methionine in a free form) is generally 0.1 mg/L/day to 55000 mg/L/day, preferably 1 mg/L/day to 1000 mg/L/day, more preferably 1 mg/L/day to 100 mg/L/day, and the concentration of arginine (concentration after conversion to arginine in a free form) is generally 0.1 mg/L/day to 150000 mg/L/day, preferably 1 mg/L/day to 2000 mg/L/day, more preferably 1 mg/L/day to 200 mg/L/day.

In one embodiment, moreover, choline (e.g., chloride choline, etc.) may be further added to the medium of the present invention. In the method of the present invention, the amount of choline (content after conversion to free form) to be added to the medium is generally 0.01 to 1000000 mg/L/day, preferably 0.1 to 1000 mg/L/day, more preferably 1 to 100 mg/L/day (for example,
[1] 1 to 100 mg/L/day, 10 to 100 mg/L/day, 20 to 100 mg/L/day, 30 to 100 mg/L/day, 40 to 100 mg/L/day, 50 to 100 mg/L/day, 60 to 100 mg/L/day, 70 to 100 mg/L/day, 80 to 100 mg/L/day, 90 to 100 mg/L/day;
[2] 1 to 90 mg/L/day, 10 to 90 mg/L/day, 20 to 90 mg/L/day, 30 to 90 mg/L/day, 40 to 90 mg/L/day, 50 to 90 mg/L/day, 60 to 90 mg/L/day, 70 to 90 mg/L/day, 80 to 90 mg/L/day;
[3] 1 to 80 mg/L/day, 10 to 80 mg/L/day, 20 to 80 mg/L/day, 30 to 80 mg/L/day, 40 to 80 mg/L/day, 50 to 80 mg/L/day, 60 to 80 mg/L/day, 70 to 80 mg/L/day;
[4] 1 to 70 mg/L/day, 10 to 70 mg/L/day, 20 to 70 mg/L/day, 30 to 70 mg/L/day, 40 to 70 mg/L/day, 50 to 70 mg/L/day, 60 to 70 mg/L/day;
[5] 1 to 60 mg/L/day, 10 to 60 mg/L/day, 20 to 60 mg/L/day, 30 to 60 mg/L/day, 40 to 60 mg/L/day, 50 to 60 mg/L/day;
[6] 1 to 50 mg/L/day, 10 to 50 mg/L/day, 20 to 50 mg/L/day, 30 to 50 mg/L/day, 40 to 50 mg/L/day;
[7] 1 to 40 mg/L/day, 10 to 40 mg/L/day, 20 to 40 mg/L/day, 30 to 40 mg/L/day;
[8] 1 to 30 mg/L/day, 10 to 30 mg/L/day, 20 to 30 mg/L/day;
[9] 1 to 20 mg/L/day, 10 to 20 mg/L/day).

In one embodiment, the pluripotent stem cell may be an embryonic stem cell (ES cell) or an iPS cell, preferably an iPS cell.

As described above, pluripotent stem cells obtained by suspension culture using the medium composition of the present invention not only proliferate efficiently, but also have a good state in maintainingan undifferentiated state. Therefore, the method of the present invention can be paraphrased as a method for promoting proliferation and/or maintaining an undifferentiated state of pluripotent stem cells.

In the method of the present invention, the culture conditions are not particularly limited, and a method known per se may be selected according to the cell type, cell density, culture method (adhesion culture/suspension culture, etc.), and the like. For example, the culture temperature may be generally 25°C to 39°C, preferably 33°C to 39°C. The carbon dioxide concentration may be generally 4% by volume to 10% by volume, preferably 4% by volume to 6% by volume. The oxygen concentration may be generally 1% by volume to 25% by volume, preferably 4% by volume to 20% by volume.

The present invention is explained in more detail in the following Examples; however, the present invention is not limited by these Examples.

### [Example]

### Example

In the following Examples, the proliferation effect and undifferentiated state maintaining ability of induced pluripotent stem cells (iPS cells) by bFGF were evaluated. As the iPS cell, 1210B2 strain purchased from iPS Academia Japan was used. In addition, as a medium for iPS cells, a commercially available StemFit AK03N (Ajinomoto Co., Inc.) or StemFit Basic03 (Ajinomoto Co., Inc.) was used.

### [Example 1] Effect of iPS cell proliferation promotion by enriching bFGF using suspension culture system

Using a 30 mL single use bioreactor for iPS cells (ABLE: BWV-S03A), iPS cell 1210B2 strain was seeded in StemFit AK03N+10 µM Y-27632 (Wako: 034-24024) at a cell density of 6×10⁵ cells/mL and the cells were cultured with stirring in a CO₂ incubator under the conditions of 37°C, CO₂ concentration=5%, stirring speed=120 rpm. On the second day of seeding, 70% of the medium was replaced with StemFit AK03N. On the third day of seeding, the cell suspension (10 mL) was resuspended in fresh StemFit Basic03+100 ng/mL bFGF (Peprotech) or StemFit Basic03+300 ng/mL bFGF. The cells were transferred to a bioreactor ambr15 (sartorius: 001-0881), and stirring culture was continued under the conditions of 37°C, pH=7.2, dissolved oxygen concentration=20%, stirring speed=300 rpm. 70% of each medium was exchanged twice a day, and 40 mg/L/day Trp (Ajinomoto Co., Inc.), 40 mg/L/day Ser (Ajinomoto Co., Inc.), 40 mg/L/day Cys (Japan protein), 40 mg/L/day Met (Ajinomoto Co., Inc.), 160 mg/L/day Arg (Ajinomoto Co., Inc.), 4 g/L/day D-glucose (Nacalai Tesque:16806-25) were further added to both groups. After the 5th day of culture, the number of viable cells was measured using Vi-CELL^{™} XR (Beckman Coulter), a live/dead cell autoanalyzer. StemFit Basic03 does not contain bFGF.

The verification results of the influence of bFGF concentration on the proliferation of iPS cells in n=1 are shown in Fig. 1. The proliferation of iPS cells was promoted by increasing the concentration of bFGF in the medium from 100 ng/mL to 300 ng/mL.

### [Example 2] Effect of maintaining undifferentiated state of iPS cell by enriching bFGF using suspension culture system

Using a 30 mL single use bioreactor for iPS cells, iPS cell 1210B2 strain was seeded in StemFit AK03N+10 µM Y-27632 at a cell density of 6×10⁵ cells/mL and the cells were cultured with stirring in a CO₂ incubator under the conditions of 37°C, CO₂ concentration=5%, stirring speed=120 rpm. On the second day of seeding, 70% of the medium was replaced with StemFit AK03N. On the third day of seeding, the cell suspension (10 mL) was resuspended in fresh StemFit Basic03+100 ng/mL bFGF (Peprotech) or StemFit Basic03+150 ng/mL bFGF. The cells were transferred to a bioreactor ambr15, and stirring culture was continued under the conditions of 37°C, pH=7.2, dissolved oxygen concentration=20%, stirring speed=300 rpm. 70% of each medium was exchanged once a day, and 40 mg/L/day Trp, 40 mg/L/day Ser, 40 mg/L/day Cys, 40 mg/L/day Met, 160 mg/L/day Arg, 4 g/L/day D-glucose were further added to both groups. On the 10th day of culture, the cells were recovered, and CD30, an iPS cell marker, was stained as follows.

To stain CD30, 2×10⁵ cells were dispensed into 1.5 mL eppen tube. After centrifugation (400×g, 4°C, 5 min), the supernatant was removed and 20 µL of PBS(-) (Nacalai Tesque: 14249-24) containing 0.2% BSA (Nacalai Tesque: 01281-84) (hereinafter 0.2% BSA-PBS) and supplemented with 20% of PE Mouse anti-CD30 (BD Biosciences: 550041) was added, and the mixture was homogenized by pipetting 5 to 10 times and left standing for 20 min under shading at 4°C. 0.5 mL of 0.2% BSA-PBS was added, and the mixture was centrifuged (400×g, 4°C, 5 min). The supernatant was removed, 0.2% BSA-PBS (300 µl) was added, and the mixture was homogenized by pipetting 5 to 10 times and transferred to a 5 mL round tube with a 35 µm cell strainer.

The expression rate of CD30 stained as described above was measured using Attune NxT Flow Cytometer (Thermo Fisher Scientific). The verification results of the influence of the amount of bFGF added on the expression of CD30 in n=1 are shown in Fig. 2. The expression of CD30 in the iPS cells was maintained as high as about 80% on the 10th day of culture, by increasing the concentration of bFGF in the medium from 100 ng/mL to 150 ng/mL.

### [Example 3] Effect of maintaining undifferentiated state of iPS cell by using stabilized bFGF

Using a 30 mL single use bioreactor for iPS cells, iPS cell 1210B2 strain was seeded in StemFit AK03N+10 uM Y-27632 at a cell density of 6×10⁵ cells/mL and the cells were cultured with stirring in a CO₂ incubator under the conditions of 37°C, CO₂ concentration=5%, stirring speed=120 rpm. On the second day of seeding, 70% of the medium was replaced with StemFit AK03N. On the third day of seeding, the cell suspension (10 mL) was resuspended in fresh StemFit Basic03+100 ng/mL bFGF (Peprotech) or StemFit Basic03+100 ng/mL Heat Stable Recombinant Human bFGF (Thermo Fisher Scientific) (hereinafter stabilized bFGF). The cells were transferred to a micro bioreactor ambr15, and stirring culture was continued under the conditions of 37°C, pH=7.2, dissolved oxygen concentration=20%, stirring speed=300 rpm. 70% of the medium was exchanged once or twice a day in the case of the medium of StemFit Basic03+100 ng/mL bFGF and once a day in the case of the medium of StemFit Basic03+100 ng/mL stabilized bFGF, and 40 mg/L/day Trp, 40 mg/L/day Ser, 40 mg/L/day Cys, 40 mg/L/day Met, 160 mg/L/day Arg, 4 g/L/day D-glucose were further added to both groups. On the 10th day of culture, the cells were recovered, and CD30, an iPS cell marker, was stained as follows. That is, 2×10⁵ cells were dispensed into 1.5 mL eppen tube. After centrifugation (400×g, 4°C, 5 min), the supernatant was removed and 20 uL of PBS(-) (Nacalai Tesque: 14249-24) containing 0.2% BSA (Nacalai Tesque: 01281-84) (hereinafter 0.2% BSA-PBS) and supplemented with 20% of PE Mouse anti-CD30 (BD Biosciences: 550041) was added, and the mixture was homogenized by pipetting 5 to 10 times and left standing for 20 min under shading at 4°C. 0.5 mL of 0.2% BSA-PBS was added, and the mixture was centrifuged (400×g, 4°C, 5 min). The supernatant was removed, 0.2% BSA-PBS (300 ul) was added, and the mixture was homogenized by pipetting 5 to 10 times and transferred to a 5 mL round tube with a 35 um cell strainer. The expression rate of CD30 stained as described above was measured using Attune NxT Flow Cytometer (Thermo Fisher Scientific). The verification results of the influence of the stabilized bFGF on the expression of CD30 in duplicate are shown in Fig. 3. It was shown that the expression of CD30 was maintained by using stabilized bFGF even though the medium was changed once a day. In other words, it was shown that the use of stabilized bFGF instead of adding a large amount of bFGF is also effective.

### [Example 4] Evaluation of differentiation potency of iPS cell cultured using a bFGF-enriched medium

### 1. high density and undifferentiated state maintenance culture of iPS cells

Using a 30 mL single use bioreactor for iPS cells (ABLE: BWV-S03A), iPS cell 1210B2 strain was seeded in StemFit (registered trade mark) AK03N+10 uM Y-27632 (Wako: 034-24024) at a cell density of 6×10⁵ cells/mL and the cells were cultured with stirring in a CO₂ incubator under the conditions of 37°C, CO₂ concentration=5%, stirring speed=120 rpm. On the second day of seeding, 70% of the medium was replaced with StemFit (registered trade mark) AK03N. On the third day of seeding, the cell suspension (10 mL) was resuspended in fresh StemFit (registered trade mark) AK03N+10 mg/L Choline Chloride (FUJIFILM Wako Pure Chemical Corporation)+200 ng/mL bFGF (Peprotech). The cells were transferred to a micro bioreactor ambr15 (sartorius: 001-0881) in duplicate, and stirring culture was continued under the conditions of 37°C, pH=7.2, dissolved oxygen concentration=4%, stirring speed=300 rpm. 70% of the medium was exchanged twice a day with StemFit (registered trade mark) AK03N+10 mg/L Choline Chloride+200 ng/mL bFGF, and 40 mg/L/day Trp (Ajinomoto Co., Inc.), 40 mg/L/day Ser (Ajinomoto Co., Inc.), 40 mg/L/day Cys (Japan protein), 40 mg/L/day Met (Ajinomoto Co., Inc.), 160 mg/L/day Arg (Ajinomoto Co., Inc.), 18.6 mg/L/day His (Ajinomoto Co., Inc.), 43.7 mg/L/day Ile (Ajinomoto Co., Inc.), 47.3 mg/L/day Leu (Ajinomoto Co., Inc.), 73.1 mg/L/day Lys HCl (Ajinomoto Co., Inc.), 28.4 mg/L/day Phe (Ajinomoto Co., Inc.), 42.3 mg/L/day Val (Ajinomoto Co., Inc.), 4 g/L/day D-glucose (Nacalai Tesque) were further added to both groups. On the 7th day of culture, the number of viable cells was quantified using Vi-CELL^{™} XR (Beckman Coulter), a live/dead cell autoanalyzer, and the expression rate of CD30, an iPS cell marker, was measured.

The results relating to the proliferation of iPS cells and undifferentiation marker are shown in Figs. 4 and 5. As shown in Figs. 1 and 2, high density culture at 8.8 to 9.7×10^6 cells/mL could be realized while maintaining an undifferentiated state of iPS cells by culturing the iPS cells in a medium containing bFGF at a high concentration.

### 2. Induction of differentiation of iPS cell into Paraxial Mesoderm (PM)

The duplicated cell suspensions cultured in the above-mentioned 1. were combined into one sample, and the cells were induced to differentiate into PM. Specifically, they were resuspended in a fresh differentiation 1 medium (AK02N - Liquid A (Ajinomoto Co., Inc.) + 20% StemFit For Differentiation (Ajinomoto Co., Inc.)+10 uM CHIR99021 (FUJIFILM Wako Pure Chemical Corporation) + 30 ng/mL activin A (Ajinomoto Co., Inc.) + 100 ng/mL bFGF+300 nM LDN-193189 (FUJIFILM Wako Pure Chemical Corporation)) and stirring culture was continued under the conditions of 37°C, pH=7.2, dissolved oxygen concentration=20%, stirring speed=300 rpm. After 12 hr, 70% of the medium was exchanged with differentiation 1 medium, and 6 hr later, 40 mg/L/day Trp, 40 mg/L/day Ser, 40 mg/L/day Cys, 40 mg/L/day Met, 160 mg/L/day Arg, 18.6 mg/L/day His, 43.7 mg/L/day Ile, 47.3 mg/L/day Leu, 73.1 mg/L/day Lys HCl, 28.4 mg/L/day Phe, 42.3 mg/L/day Val, 4 g/L/day D-glucose were further added. After 6 hr, the cell suspension (10 mL) was resuspended in a fresh differentiation 2 medium (AK02N - Liquid A +20% StemFit For Differentiation+5 uM CHIR99021+10 uM SB431542 (Stemgent)+100 ng/mL bFGF+300 nM LDN-193189), and culture was continued. After 12 hr, 70% of the medium was exchanged with differentiation 2 medium, and 6 hr later, 40 mg/L/day Trp, 40 mg/L/day Ser, 40 mg/L/day Cys, 40 mg/L/day Met, 160 mg/L/day Arg, 18.6 mg/L/day His, 43.7 mg/L/day Ile, 47.3 mg/L/day Leu, 73.1 mg/L/day Lys HCl, 28.4 mg/L/day Phe, 42.3 mg/L/day Val, 4 g/L/day D-glucose were added. After 6 hr, the number of viable cells and the expression rate of PM marker DLL1 were measured.

The results relating to the cell proliferation associated with differentiation into PM and differentiation marker are shown in Figs. 6 and 7. The iPS cells cultured in a medium containing bFGF at a high concentration could be highly efficiently differentiated into PM in a high density state exceeding 1.0×10^7 cells/mL.

### [Industrial Applicability]

According to the present invention, cells can be prepared extremely efficiently. According to the present invention, moreover, high quality pluripotent stem cells that maintain an undifferentiated state well can be prepared highly efficiently.

This application is based on a patent application No. 2020-003958 filed in Japan (filing date: January 14, 2020), the contents of which are incorporated in full herein.

## Claims

1. A medium composition for cell culture, comprising a basic fibroblast growth factor (bFGF) at not less than 150 ng/mL, or a stabilized bFGF at a concentration that affords an effect equivalent to that of bFGF at said concentration.

2. The medium composition according to claim 1, wherein the composition is for use in suspension culture.

3. The medium composition according to claim 1 or 2, wherein the composition is for use in high density culture.

4. The medium composition according to claim 3, wherein cells to be subjected to suspension culture have a cell density of not less than 6.0×10⁵ cells/mL.

5. The medium composition according to any one of claims 1 to 4, wherein the cell is a pluripotent stem cell, an adult stem cell, or a progenitor cell.

6. A method for culturing a cell, comprising suspension culture of the cell in the medium composition according to claim 1.

7. The method according to claim 6, wherein the medium comprises bFGF at 10 to 1000 ng/mL/day, or stabilized bFGF at a concentration that affords an effect equivalent to that of bFGF at said concentration.

8. The method according to claim 6 or 7, wherein pluripotent stem cells to be subjected to suspension culture have a cell density of not less than 6.0×10⁵ cells/mL.

9. The method according to any one of claims 6 to 8, wherein the cell is a pluripotent stem cell, an adult stem cell, or a progenitor cell.
